# EUROPÄISCHE PATENTSCHRIFT

(11) **EP 1 716 092 B1**
(45) Veröffentlichungstag und Bekanntmachung des Hinweises auf die Patenterteilung: **08.09.2010**
(21) Anmeldenummer: 05715257.1
(22) Anmeldetag: 04.02.2005
(51) Int. Cl.: C07C 45/65, C07C 47/21

(54) **VERFAHREN ZUR HERSTELLUNG VON 2,7-DIMETHYL-OCTA-2,4,6-TRIENDIAL**
METHOD FOR PRODUCING 2,7-DIMETHYL-OCTA-2,4,6-TRIENEDIAL
PROCEDE POUR PREPARER DU 2,7-DIMETHYL-OCTA-2,4,6-TRIENEDIAL

(30) Priorität: 10.02.2004 DE 102004006579
(43) Veröffentlichungstag der Anmeldung: 02.11.2006
(73) Patentinhaber: BASF SE, 67056 Ludwigshafen (DE)
(72) Erfinder: ERNST, Hansgeorg, 67346 Speyer (DE); HENRICH, Klaus, 67454 Hassloch (DE); KELLER, Andreas, 67346 Speyer (DE)
(86) Internationale Anmeldenummer: PCT/EP2005/001138
(87) Internationale Veröffentlichungsnummer: WO 2005/077874

(56) Entgegenhaltungen:
- CH-A- 321 106

## Beschreibung

Die vorliegende Erfindung betrifft ein verbessertes Verfahren zur Herstellung von 2,7-Dimethyl-octa-2,4,6-triendial der Formel I, im folgenden als C₁₀-Dial bezeichnet.

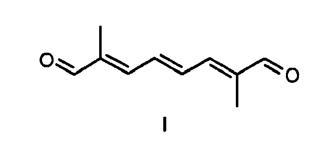

C₁₀-Dial I wird als wichtiges Zwischenprodukt für technische Verfahren zur Herstellung von Carotinoiden z.B. von Lycopin, β-Carotin und Astaxanthin benötigt (siehe: "Carotenoids", Vol. 2, "Synthesis", Seite 7 ff., Seite 92 ff. und Seite 274 ff.; Birkhäuser Verlag, 1996).

Für ein wirtschaftliches Herstellverfahren dieser begehrten Pigmente ist daher ein guter Zugang zu I von besonderer Bedeutung.

Verschiedene Herstellverfahren für C₁₀-Dial sind bekannt (siehe: "Carotenoids", Vol. 2, Seite 117/118), darunter auch die doppelte Enolether-Kondensation von Butendial-Bisacetalen der Formel II mit Propenylethern der Formel III zu einer Hexa-alkoxydimethyl-octen-Zwischenstufe der Formel IV, gefolgt von einer doppelten Acetalspaltung zu einem Dialkoxy-dial der Formel V und einer doppelten Alkanoleliminierung. 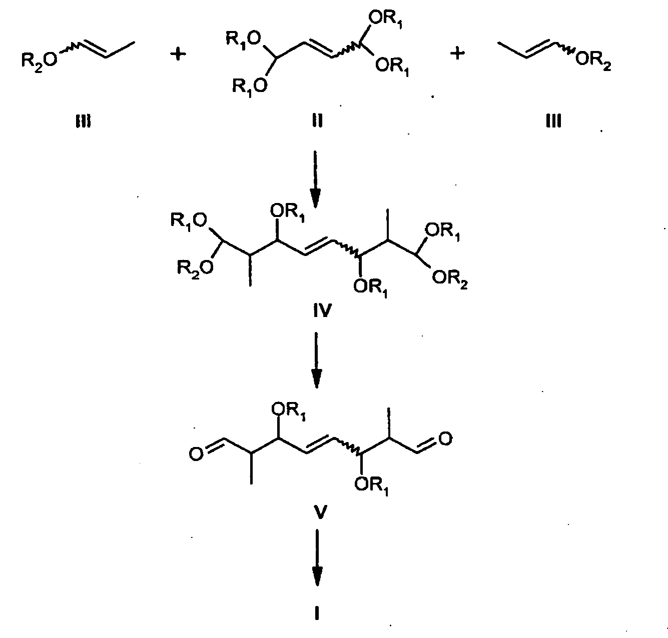 Für die Durchführung dieser Synthesesequenz werden in der Literatur verschiedene Bedingungen genannt. Gemäß CH-PS 321106 wird im ersten Schritt Butendial-bisacetal II in Gegenwart eines sauren Katalysators mit einem Propenylether III kondensiert, wobei Bortrifluorid-Etherat, ZnCl₂, TiCl₄, AlCl₃ und SnCl₄ besonders hervorgehoben werden. Dieser Prozess wird in Substanz, d.h. ohne Anwesenheit eines zusätzlichen Lösungsmittels, durchgeführt. Im nächsten Schritt werden die Acetalgruppen des Zwischenprodukts IV im sauren Milieu hydrolysiert, wobei unter gleichzeitiger Eliminierung von R₁OH aus den Positionen 2,3 und 6,7 das Endprodukt I gebildet wird. Als besonders vorteilhaft wird der Zusatz eines mit Wasser mischbaren Lösungsmittels wie Dioxan, THF oder Ethylenglycoldimethylether in Kombination mit wässriger Phosphorsäure genannt. Es wird eine Ausbeute von 56 % d. Th. angegeben.

Alternativ kann die Umsetzung von Verbindung IV zum C₁₀-Dial I gemäß CH-PS 321106 auch durch mehrstündiges Erhitzen mit wässriger Essigsäure durchgeführt werden. C₁₀-Dial wird anschließend durch Verdünnen mit Wasser ausgefällt.

Gemäß J. Gen. Chem. USSR, 34, 64 f. (1964) wird die oben genannte Enoletherkondensation der Verbindungen II und III (R₁ = R₂ = CH₃) in Gegenwart von ZnCl₂ + BF₃-Etherat ohne Zusatz eines Lösungsmittels durchgeführt. Die Hexamethoxyverbindung IV wird in 71 %iger Ausbeute destillativ isoliert. Die Überführung von IV zum C₁₀-Dial I wird durch mehrstündiges Erhitzen in 6 %iger wässriger Phosphorsäure unter simultanem Abdestillieren von gebildetem Methanol durchgeführt. Es wird eine Ausbeute von 65 % d. Th. (bezogen auf Verbindung IV) erzielt. Die Gesamtausbeute von 46 % d. Th. (bezogen auf Verbindung II) ist zudem für ein wirtschaftliches Verfahren nicht ausreichend.

Unter ganz ähnlichen Bedingungen wird die Enoletherkondensation (mit R₁ = CH₃; R₂= C₂H₅) in "Carotenoids", Vol. 2, "Synthesis", Seiten 301/302, (Birkhäuser Verlag,1996) beschrieben. Nach basischer Aufarbeitung wird - ohne weitere Reinigung - eine Rohausbeute an IV von 98% d. Th. angegeben. Die Schritte Hydrolyse und Eliminierung werden durch Erhitzen von IV in 90 %iger wässriger Essigsäure unter Zusatz von 15 % Natriumacetat durchgeführt. Nach Zugabe von Wasser fällt in 70 %iger Ausbeute ein braunes Produkt mit unbefriedigender Reinheit aus, so dass aus Toluol unter Zusatz von Aktivkohle umkristallisiert werden muss. Die Gesamtausbeute an C₁₀-Dial I beträgt nur etwa 57 % d. Th. (bezogen auf Verbindung II).

Gemäß EP 784042 wird die Enoletherkondensation (mit R₁ = R₂ = CH₃) in Substanz in Gegenwart von Hydrogen-bis(oxalato)borat als saurem Katalysator in Abwesenheit eines Lösungsmittels beschrieben. Zur Hydrolyse der Acetalschutzgruppen wird das Reaktionsgemisch in Substanz unter Abdestillieren von entstandenem Methanol erhitzt. Nach Alkalisch-Stellung des Reaktionsgemischs mit 15 %iger Natronlauge fällt C₁₀-Dial I aus. Es wird keine Angabe über die Reinheit des Produkts gemacht; nach LC-Analyse wird eine Ausbeute von 67 % ermittelt.

Sämtliche der eingangs beschriebenen Verfahren liefern nur eine unbefriedigende Gesamtausbeute an C₁₀-Dial. Zudem ist die Reinheit des Produkts, das aus dem wässrigen Milieu ausfällt, nicht ausreichend, um es direkt in die Synthese von Carotinoiden einsetzen zu können, so dass eine zusätzliche Umkristallisation häufig erforderlich ist.

Es war daher Aufgabe der vorliegenden Erfindung, ein Verfahren zur Herstellung von 2,7-Dimethyl-octa-2,4,6-triendial in hoher Ausbeute und in hoher Reinheit bereitzustellen.

Diese Aufgabe wurde gelöst durch ein Verfahren zur Herstellung von 2,7-Dimethyl-octa-2,4,6-triendial der Formel I, 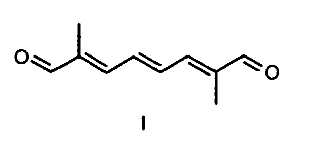 durch
a) doppelte Enoletherkondensation eines Butendial-bisacetals der Formel II 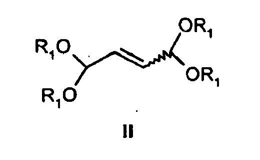 mit einem Enolether der Formel III, 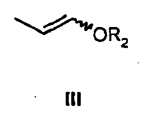 in Gegenwart eines Lewis-sauren Katalysators zu einem Kondensationsprodukt der Formel IV, 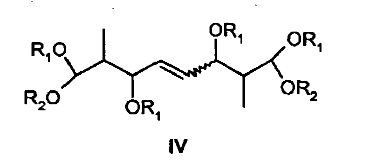 wobei die Reste R₁ und R₂ der Formeln II bis IV unabhängig voneinander für C₁-C₆-Alkyl stehen;
b) Hydrolyse der Acetalgruppen von IV durch Zugabe einer wässriger Säure unter Bildung des Dialdehyds der Formel V; 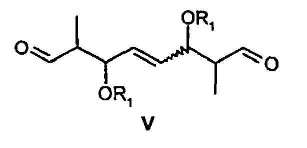
c) Überführung von V in den Dialdehyd I durch Reaktion mit einer wässrigen Base
   und
d) Kristallisation von I aus dem Reaktionsgemisch,
**dadurch gekennzeichnet, dass** man die Verfahrensschritte a) bis d) in Gegenwart eines inerten, mit Wasser nicht mischbaren organischen Lösungsmittels durchführt.

Als Alkylreste für R₁ und R² seien lineare oder verzweigte C₁-C₆-Alkylketten, z.B. Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl genannt. Bevorzugte Alkylreste sind Methyl, Ethyl, n-Propyl und 1-Methylethyl, besonders bevorzugt Methyl und Ethyl, ganz besonders bevorzugt Methyl.

Die räumliche Anordnung der Substituenten an den C-C-Doppelbindungen der Verbindungen II, III und IV sowie die Konfiguration der mittleren C-C-Doppelbindung der Verbindung V ist für die Durchführbarkeit des erfindungsgemäßen Verfahrens nicht relevant. Es kann sich dabei sowohl um isomerenreine cis- bzw. trans-Verbindungen als auch um cis-trans-Gemische handeln. In der Regel werden im Rahmen der vorliegenden Erfindung cis-trans-Gemische der Verbindungen II bis V eingesetzt.

Bei dem erfindungsgemäß hergestellten 2,7-Dimethyl-octa-2,4,6-triendial der Formel I liegen die C-C-Doppelbindungen überwiegend in der all-trans-Konfiguration vor. Der Anteil an all-trans C₁₀-Dial liegt dabei in der Regel im Bereich zwischen 85 und 95 %.

Unter "inerten, mit Wasser nicht mischbaren organischen Lösungsmitteln" sind vor allem offenkettige oder cyclische Kohlenwasserstoffe wie etwa Hexan, Heptan oder Cyclohexan, aromatische Kohlenwasserstoffe wie Benzol, Toluol oder Xylol, Dialkylether wie etwa Diisopropylether, Di-n-butylether oder Methyl-tert.butylether sowie C₁ bis C₆ Alkylester von Alkancarbonsäuren wie beispielsweise Essigsäureethylester sowie Mischungen dieser Lösungsmittel zu verstehen. Vorzugsweise setzt man aromatische Kohlenwasserstoffe, besonders bevorzugt Toluol, ein.

Beim ersten Schritt des erfindungsgemäßen Verfahrens, der zweifachen Enoletherkondensation von II mit III, geht man in der Regel so vor, dass man Verbindung 11 zusammen mit einem sauren Katalysator vorlegt. Die Konzentration an II ist unkritisch; sie liegt in der Regel bei 5 bis 15 Gew.-%, bevorzugt bei etwa 10 Gew.%, bezogen auf das Gesamtgewicht der Lösung.

Als Katalysatoren sind generell die in der Literatur für Enoletherkondensationen dieser Art genannten Lewis-Säuren geeignet (s.u.a. "Carotenoids", Vol. 2, p. 29/30); bevorzugt zu nennen sind BF₃-Etherat, ZnCl₂ und FeCl₃, sowie deren Mischungen. Besonders bevorzugt ist wasserfreies FeCl₃.

Die Katalysatoren werden in einer Menge von 0,05 bis 10 mol-%, bevorzugt in einer Menge von 0,1 bis 5 mol-% (bezogen auf Verbindung II) eingesetzt. Die Reaktionstemperatur bei der Enoletherkondensation liegt im Bereich von etwa 0°C bis 50°C, vorzugsweise bei 20°C bis 30°C.

Die Mengen an eingesetztem Enolether III liegen in der Regel im Bereich von 1,9 bis 2,3 mol, bevorzugt im Bereich von 2 bis 2,2 mol, besonders bevorzugt bei 2,1 mol pro mol eingesetztem Butendial-bisacetal II.

Die erfindungsgemäße Enoletherkondensation wird vorteilhafterweise so durchgeführt, dass man das Butendial-bisacetal 11 zusammen mit der Lewissäure vorlegt und den Enolether III kontinuierlich über mehrere Stunden, bevorzugt 3 bis 6 h zudosiert.

Ein Vorteil des erfindungsgemäßen Verfahrens besteht darin, dass keine Aufarbeitung des Reaktionsgemischs der Enoletherkondensation erforderlich ist. Vielmehr kann die nachfolgende Acetalhydrolyse mit dem nicht aufgearbeiteten Reaktionsgemisch der ersten Stufe durchgeführt werden.

Als saure Katalysatoren für die Stufe b) sind wässrige Lösungen anorganischer Säuren wie etwa Schwefelsäure, Salpetersäure, Phosphorsäure, Borsäure oder Halogenwasserstoffsäuren sowie saure Salze mehrbasiger Säuren wie etwa Alkali- oder Erdalkalimetall-hydrogensulfate geeignet. Außerdem sind wässrige Lösungen von Salzen mit saurem pH für die Acetalspaltung geeignet, wie z.B. wässrige Lösungen von FeCl₃ oder ZnCl₂, so dass gegebenenfalls das Reaktionsgemisch der Enoletherkondensation ohne weiteren Zusatz einer Säure lediglich mit Wasser versetzt werden muss. Neben diesen anorganischen Säuren sind auch wässrige Lösungen organischer Säuren wie etwa Sulfonsäuren (Methansulfonsäure, Benzolsulfonsäure, Toluolsulfonsäure) sowie anderer organischer Säuren wie etwa Citronensäure, Oxalsäure, Ameisensäure und Niederalkancarbonsäuren wie z.B. Essigsäure für diese Acetalspaltung geeignet.

Bevorzugte saure Katalysatoren sind Schwefelsäure, Salpetersäure, Phosphorsäure oder Halogenwasserstoffsäuren oder deren Mischungen, besonders bevorzugt verwendet man Schwefelsäure.

Die Konzentration der Säure in der wässrigen Lösung liegt im Bereich von etwa 0,1 bis 20 Gew.%, bevorzugt bei etwa 2 bis 10 Gew.%, die Reaktionstemperatur liegt im Bereich von 20°C bis zur jeweiligen Siedetemperatur des eingesetzten Lösungsmittels, bevorzugt im Bereich von 30°C bis 90°C, besonders bevorzugt im Bereich von 70°C bis 90°C.

Die Acetalspaltung kann einstufig erfolgen, wobei der gebildete Alkohol dem Acetalspaltungs-/Acetalisierungsgleichgewicht vorzugsweise durch Destillation bei Normaldruck oder vermindertem Druck entzogen wird. Alternativ kann der entstandene Alkohol auch durch Extraktion in die Wasserphase abgetrennt werden. Hier ist gegebenenfalls eine 2 bis 3-stufige Fahrweise zur Vervollständigung des Umsatzes vorteilhaft.

An die Acetalspaltung (Umsetzung von Verbindung IV zu Verbindung V) schließt sich die Eliminierungsreaktion c) zum C₁₀-Dial an. Dazu wird zu der Lösung des Dialkoxydials V eine Base gegeben. Die saure wässrige Phase der Acetalspaltung kann dabei direkt alkalisch gestellt werden, wird aber in der bevorzugten Ausführungsform des erfindungsgemäßen Verfahrens vor der Basenzugabe abgetrennt.

Als Basen für die Eliminierungsreaktion sind wässrige Lösungen von Alkali- und Erdalkalimetall-hydroxiden, - carbonaten und hydrogencarbonaten, Trialkylaminen wie etwa Triethylamin oder Alkali- bzw- Erdalkalimetallsalzen organischer Säuren wie etwa Acetate, Formiate oder Oxalate geeignet. Bevorzugt sind wässrige Lösungen von Natriumhydroxid, Natriumcarbonat oder Natriumhydrogencarbonat oder Mischungen davon, besonders bevorzugt ist eine wässrige Lösung von Natriumhydrogencarbonat.

Die Konzentration der Base in der wässrigen Lösung beträgt etwa 0,2 bis 15 Gew.-%, vorzugsweise etwa 1 bis10 Gew.-%. Die Reaktionstemperatur liegt im Bereich von 20°C bis zur jeweiligen Siedetemperatur des eingesetzten Lösungsmittels, bevorzugt im Bereich von 30°C bis 90°C, besonders bevorzugt im Bereich von 70°C bis 90°C. Der entstandene Alkohol kann gewünschtenfalls bei Normaldruck oder vermindertem Druck abdestilliert werden.

Nach Abschluss der Eliminierungsreaktion wird die wässrige Phase abgetrennt. Die organische Phase kann gewünschtenfalls zur Entfernung wasserlöslicher Verunreinigungen mit Wasser gewaschen werden.

Das aus der organischen Phase ausgefallene Produkt kann in an sich bekannter Weise durch Filtration isoliert werden. Noch in der Mutterlauge gelöstes Wertprodukt kann durch weiteres Aufkonzentrieren und nochmalige Kristallisation, besonders vorteilhaft aber durch Rückführung der Mutterlauge unter partieller Ausschleusung (Ausschleusrate etwa 5 bis 15%, bevorzugt 10%) gewonnen werden.

Man erhält so den C₁₀-Dial I in einer Ausbeute von mindestens 80% d. Th. (bezogen auf Verbindung II) und mit einer Reinheit von über 99,5% (nach GC). Dieses Produkt ist ohne weiteren Reinigungsschritt für die eingangs genannten Carotinoid-Endstufen geeignet.

In einer anderen Ausführungsform des erfindungsgemäßen Verfahrens kann die Base in Substanz zur Lösung von V im jeweiligen inerten organischen Lösungsmittel zugeben werden. Als Basen kommen hier neben Trialkylaminen wie z.B. Triethylamin, Tripropylamin oder Tributylamin auch Alkali- oder Erdalkalimetall-hydroxide, -carbonate und hydrogenacarbonate sowie Salze organischer Säuren wie z.B. Acetate, Formiate oder Oxalate. Diese Basen können in katalytischer Menge, bevorzugt aber in molarer Menge (bezogen auf Verbindung V) oder in mehrfachem molaren Überschuss eingesetzt werden. Nach Abschluss der Eliminierung wird der basische Eliminierungskatalysator durch eine oder mehrere Waschungen mit Wasser ausgewaschen.

Anhand der folgenden Beispiele soll das erfindungsgemäße Verfahren näher beschrieben werden.

### Beispiel 1

179,1 g (1,013 mol) Butendial-bisacetal II (R₁ = Methyl) und 0,35 g (2,16 mmol) wasserfreies Eisen-(III)-chlorid wurden in 2 I Toluol vorgelegt. Innerhalb von 4 h ließ man 147,6 g (2,026 mol) 1-Methoxypropen III (R₂ = CH₃) zulaufen. Dabei wurde die Reaktionstemperatur bei 25°C gehalten. Nach Zulaufende wurde 2 h bei + 25°C nachgerührt. Dann gab man 400 g 2 %ige wässrige Schwefelsäure zu, erwärmte auf 80°C und rührte 4 h bei dieser Temperatur nach. Dann wurde die Wasserphase abgetrennt. Man gab wiederum 400 ml 2 %ige Schwefelsäure zu, rührte 2 h bei 80°C nach und trennte die Wasserphase ab. Diese Operation wurde noch einmal wiederholt. Anschließend gab man 1000 g einer 5 %igen Natriumhydrogencarbonat-Lösung zu und rührte 3 h bei einer Temperatur von 80 bis 85°C nach. Man trennte die wässrige Unterphase ab und wusch die organische Oberphase bei 80°C mit 500 ml Wasser von 80°C. Die Wasserphase wurde abgetrennt und die organische Phase unter vermindertem Druck auf 1 I aufkonzentriert. Die konzentrierte organische Phase wurde auf 0°C abgekühlt; die entstandene Suspension von I wurde 1 h bei 0°C ausgerührt. Das Produkt wurde abfiltriert, zweimal mit je 250 ml kaltem Toluol gewaschen und im Vakuumtrockenschrank bei 50°C/20 mbar getrocknet.

| | |
|---|---|
| Auswaage: | 128,3 g C₁₀-Dial I |
| Ausbeute: | 77,3 % d. Th. |
| Fp.: | 162°C-163°C |
| Reinheit (GC): | 100 % |

Durch Aufkonzentrierung von vereinigter Mutter- und Waschlauge konnten nochmals 10,5 g (6,3 % d. Th.) I isoliert werden.

Das durch Restlöslichkeit in Mutter- und Waschlaugen noch enthaltene Wertprodukt konnte auch durch Rückführung in die Kristallisation des nächsten Ansatzes isoliert werden (s. Beispiel 2).

### Beispiel 2

98,9 g (0,55 mol) Butendial II (R₁ = Methyl) und 0,268 g (1,65 mmol) wasserfreies Eisen-(III)-chlorid wurden in 682 g Toluol vorgelegt. Innerhalb von 4 h ließ man 79,3 g (1,10 mol) 1-Methoxypropen III (R₂ = CH₃) zulaufen. Dabei wurde die Reaktionstemperatur bei 25°C gehalten. Nach Zulaufende wurde 2 h bei 25°C nachgerührt.

Man erhielt einen Reaktionsaustrag von 855 g.

Ein Anteil von 389 g (das entsprach einem Anteil von 0,455 des Gesamtumsatzes) wurde mit 125 ml 0,5 %iger Schwefelsäure versetzt. Man rührte 5 h bei 85°C nach; dabei wurde entstandenes Methanol bei Normaldruck abdestilliert.

Anschließend ließ man bei 85°C zu diesem zweiphasigen Gemisch ohne vorherige Abtrennung der schwefelsauren Wasserphase innerhalb von 1 h 110 g einer 5 %igen Natriumhydrogencarbonat-Lösung zulaufen und rührte weitere 3 h bei 85°C nach, wobei entstandenes Methanol fortlaufend bei Normaldruck abdestilliert wurde. Dann trennte man bei 80°C die wässrige Unterphase ab. Die 80°C heiße Oberphase wurde mit 75 ml Wasser von 80°C gewaschen.

Die wässrige Phase wurde abgetrennt.

Die organische Phase wurde bei 400 mbar aufkonzentriert. Dabei wurden simultan 90% der Mutterlauge sowie die gesamte Waschlauge eines identischen vorhergehenden Ansatzes zugefahren. Bei einem Endvolumen von ca. 250 ml kühlte man auf 0°C ab. Die entstandene Suspension von I wurde 1 h bei 0°C ausgerührt. Das Produkt wurde abfiltriert, zweimal mit je 50 ml kaltem Toluol gewaschen und im Trockenschrank bei 50°C/20 mbar getrocknet.

| | |
|---|---|
| Auswaage: | 34,46 g C₁₀-Dial I |
| Ausbeute: | 84,1 % d. Th. |
| Fp.: | 163°C-164°C |
| Reinheit (GC): | 100 % |

Mutterlauge und Waschlauge wurden getrennt aufgefangen. 90 % der Mutterlauge und die gesamte Waschlauge wurden in den Aufkonzentrierungsschritt eines identischen Folgeansatzes rückgeführt. 10 % der Mutterlauge wurden ausgeschleust und entsorgt.

### Beispiel 3

Eine Lösung von 80,1 g Enoletherkondensationsprodukt IV (R₁ = R₂ = CH₃) in 500 ml Toluol, die analog Beispiel 1 hergestellt wurde, wurde mit 250 ml 2,5 %iger wässriger Phosphorsäure versetzt. Man erhitzte auf eine Innentemperatur von 85 bis 90°C und destillierte über einen Zeitraum von 5 h Methanol ab. Die wässrige Unterphase wurde abgetrennt. Die organische Oberphase wurde gaschromatographisch analysiert. Sie enthielt 86,6 % Dimethoxydial V, 4,5 % Enoletherkondensationsprodukt IV und 7,2 % C₁₀-Dial I.

### Beispiel 4

Eine Lösung von 8,01 g Enoletherkondensationsprodukt IV (R₁ = R₂ = CH₃) in 50 ml Toluol, die analog Beispiel 1 hergestellt wurde, wurde mit 25 ml 10 %iger wässriger Oxalsäurelösung 1 h bei 80°C gerührt. Dann wurde die organische Phase gaschromatographisch analysiert:
89,3 % Dimethoxydial V, 1,7 % Enoletherkondensationsprodukt IV.

### Beispiel 5

Zu einer Lösung von 2,28 g (10 mmol) Dimethoxydial V in 20 ml Toluol, die analog Beispiel 1 hergestellt wurde, wurden 50,6 mg (0,5 mmol) Triethylamin zugegeben. Dann wurde das Gemisch 8 h unter Rückfluss erhitzt. Die GC-Analyse zeigte danach 98,5 % C₁₀-Dial I und 1,5 % Dimethoxydial V.

### Beispiel 6

Zu einer Lösung aus 2,86 g (12,5 mmol) Dimethoxydial V in 25 ml Toluol, die analog Beispiel 1 hergestellt wurde, wurden 2,86 g (34 mmol) festes Natriumhydrogencarbonat gegeben. Die Suspension wurde 8 h unter Rückfluss erhitzt. Die GC-Analyse zeigt danach 92 % C₁₀-Dial I und 7,5 % Dimethoxydial V.

## Patentansprüche

1. Verfahren zur Herstellung von 2,7-Dimethyl-octa-2,4,6-triendial der Formel I, 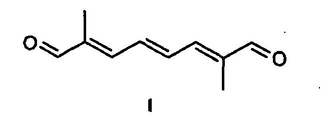 durch
a) doppelte Enoletherkondensation eines Butendial-bisacetals der Formel II 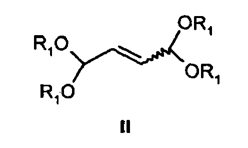 mit einem Enolether der Formel III, 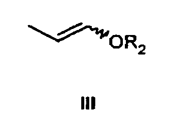 in Gegenwart eines Lewis-sauren Katalysators zu einem Kondensationsprodukt der Formel IV, 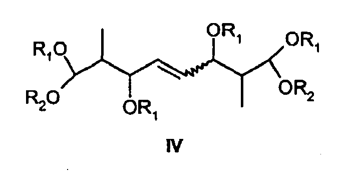 wobei die Reste R₁ und R₂ der Formeln II bis IV unabhängig voneinander für C₁-C₆-Alkyl stehen;
b) Hydrolyse der Acetalgruppen von IV durch Zugabe einer wässrigen Säure
unter Bildung des Dialdehyds der Formel V; 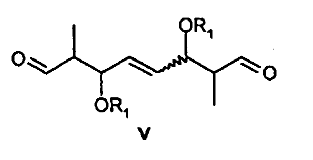
c) Überführung von V in den Dialdehyd I durch Reaktion mit einer wässrigen Base und
d) Kristallisation von I aus dem Reaktionsgemisch,
**dadurch gekennzeichnet, dass** man die Verfahrensschritte a) bis d) in Gegenwart eines inerten, mit Wasser nicht mischbaren organischen Lösungsmittels durchführt.

2. Verfahren nach Anspruch 1, **dadurch gekennzeichnet, dass** man in allen Verfahrensschritten a) bis d) Toluol als Lösungsmittel verwendet.

3. Verfahren nach Anspruch 1 oder 2, **dadurch gekennzeichnet, dass** man die doppelte Enoletherkondensation im Verfahrensschritt a) in Gegenwart von ZnCl₂, BF₃-Etherat oder FeCl₃ oder von deren Gemischen durchführt.

4. Verfahren nach Anspruch 3, **dadurch gekennzeichnet, dass** man als Lewis-sauren Katalysator wasserfreies FeCl₃ einsetzt.

5. Verfahren nach einem der Ansprüche 1 bis 4, **dadurch gekennzeichnet, dass** man für die Acetalspaltung im Verfahrensschritt b) wässrige Schwefel-, Salpeter-, Phosphor- oder Halogenwasserstoffsäure oder deren Mischungen einsetzt.

6. Verfahren nach Anspruch 5, **dadurch gekennzeichnet, dass** man wässrige Schwefelsäure verwendet.

7. Verfahren nach einem der Ansprüche 1 bis 6, **dadurch gekennzeichnet, dass** man für die Eliminierungsreaktion im Verfahrensschritt c) wässrige Lösungen von Alkali- oder Erdalkalimetall-hydroxiden, -carbonaten oder -hydrogencarbonaten einsetzt.

8. Verfahren nach Anspruch 7, **dadurch gekennzeichnet, dass** man eine wässrige Natriumhydrogencarbonatlösung verwendet.

## Claims

1. A process for preparing 2,7-dimethylocta-2,4,6-trienedial of the formula I, 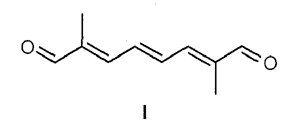 by
a) double enol ether condensation of a butenedial bisacetal of the formula II 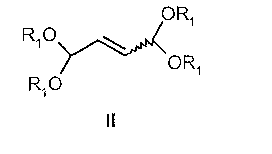 with an enol ether of the formula III, 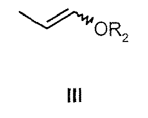 in the presence of a Lewis acid catalyst to give a condensation product of the formula IV, 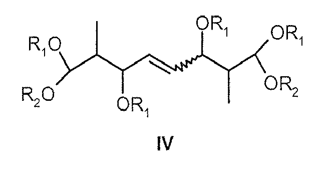 where the radicals R₁ and R₂ in formulae II to IV are independently of one another C₁-C₆-alkyl;
b) hydrolysis of the acetal groups of IV by adding an aqueous acid to form the dialdehyde of the formula V; 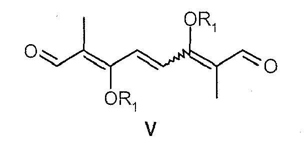
c) conversion of V into the dialdehyde I by reacting with an aqueous base and
d) crystallization of I from the reaction mixture,
wherein process steps a) to d) are carried out in the presence of an inert, water-immiscible organic solvent.

2. The process according to claim 1, wherein toluene is used as solvent in all of process steps a) to d).

3. The process according to claim 1 or 2, wherein the double enol ether condensation in process step a) is carried out in the presence of ZnCl₂, BF₃ etherate or FeCl₃ or of mixtures thereof.

4. The process according to claim 3, wherein anhydrous FeCl₃ is employed as Lewis acid catalyst.

5. The process according to any of claims 1 to 4, wherein aqueous sulfuric, nitric, phosphoric or hydrohalic acid or mixtures thereof are employed for the acetal cleavage in process step b).

6. The process according to claim 5, wherein aqueous sulfuric acid is used.

7. The process according to any of claims 1 to 6, wherein aqueous solutions of alkali metal or alkaline earth metal hydroxides, carbonates or bicarbonates are employed for the elimination reaction in process step c).

8. The process according to claim 7, wherein an aqueous sodium bicarbonate solution is used.

## Revendications

1. Procédé pour la préparation de 2,7-diméthylocta-2,4,6-triènedial de formule I 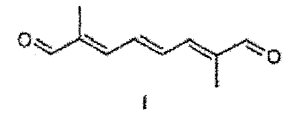 par
a) une double condensation énoléther d'un bisacétal de butènedial de formule II 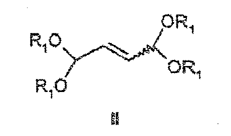 avec un énoléther de formule III, 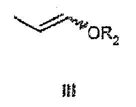 en présence d'un catalyseur acide de Lewis en un produit de condensation de formule IV, 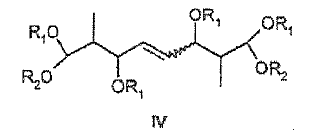 où les radicaux R₁ et R₂ des formules II à IV représentent, indépendamment l'un de l'autre, C₁-C₆-alkyle ;
b) hydrolyse des groupes acétal de IV par addition d'un acide aqueux avec formation du dialdéhyde de formule V ; 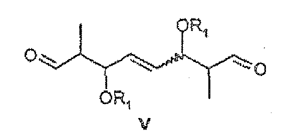
c) transformation de V en dialdéhyde I par réaction avec une base aqueuse et
d) cristallisation de I à partir du mélange réactionnel,
**caractérisé en ce qu'**on réalise les étapes de procédé a) à d) en présence d'un solvant organique inerte, non miscible à l'eau.

2. Procédé selon la revendication 1, **caractérisé en ce qu'**on utilise dans toutes les étapes de procédé a) à d) du toluène comme solvant.

3. Procédé selon la revendication 1 ou 2, **caractérisé en ce qu'**on réalise la double condensation énoléther dans l'étape de procédé a) en présence de ZnCl₂, d'éthérate de BF₃ ou de FeCl₃ ou de leurs mélanges.

4. Procédé selon la revendication 3, **caractérisé en ce qu'**on utilise comme catalyseur acide de Lewis du FeCl₃ anhydre.

5. Procédé selon l'une quelconque des revendications 1 à 4, **caractérisé en ce qu'**on utilise pour la dissociation de l'acétal dans l'étape de procédé b) de l'acide sulfurique, nitrique, phosphorique on halogénohydrique aqueux ou leurs mélanges.

6. Procédé selon la revendication 5, **caractérisé en ce qu'**on utilise de l'acide sulfurique aqueux.

7. Procédé selon l'une quelconque des revendications 1 à 6, **caractérisé en ce qu'**on utilise pour la réaction d'élimination dans l'étape de procédé c) des solutions aqueuses d'hydroxydes, de carbonates ou d'hydrogénocarbonates de métal alcalin ou alcalino-terreux.

8. Procédé selon la revendication 7, **caractérisé en ce qu'**on utilise une solution aqueuse d'hydrogénocarbonate de sodium.
